# EUROPEAN PATENT SPECIFICATION

(11) **EP 1 863 435 B1**
(45) Date of publication and mention of the grant of the patent: **02.11.2016**
(21) Application number: 06723917.8
(22) Date of filing: 22.03.2006
(51) Int. Cl.: A61K 8/14, A61K 8/55, A61K 8/37, A61Q 19/00

(54) **ENHANCED DELIVERY OF SKIN BENEFIT AGENTS**
VERBESSERTE ZUFÜHRUNG VON HAUTPFLEGEMITTELN
ADMINISTRATION AMELIOREE D'AGENTS DE SOIN POUR LA PEAU

(30) Priority: 31.03.2005 IN MU03872005; 19.07.2005 GB 0514714
(43) Date of publication of application: 12.12.2007
(73) Proprietor: Unilever PLC, London EC4Y 0DY (GB); Unilever N.V., 3013 AL Rotterdam (NL)
(72) Inventor: Bandyopadhyay, Prasun Hindustan Lever Ltd, Bangalore 560 066 (IN); Bandyopadhyay, Punam Hindustan Lever Ltd, Bangalore 560 066 (IN)
(74) Representative: Corsten, Michael Allan
(86) International application number: PCT/EP2006/002957
(87) International publication number: WO 2006/103091

(56) References cited:
- EP-A- 1 438 946
- WO-A-93/05767
- WO-A-94/06401
- WO-A-98/52927
- US-A- 4 454 159
- US-A- 4 761 407
- US-A- 4 981 681
- US-A- 5 702 714
- US-A- 5 766 628
- US-A1- 2004 076 652
- US-A1- 2004 147 534
- US-B1- 6 656 499

## Description

### Technical field

The invention relates to a system for enhanced delivery of skin benefit agents, more particularly, to a pro-vesicle for enhanced delivery of skin lightening or UV blocking agents to the skin. The invention also relates to a cosmetic composition comprising the delivery system of the invention.

### Background and Prior Art

Liposomes have been known since the 1960s and are reported to be used in various applications. Liposomes are small microencapsulates formed from certain surface active molecules, most commonly phospholipids, which in aqueous media arrange themselves into a bi-layered membrane defining a microscopic closed vesicle. Liposomes are known to have good penetration capability through the skin and therefore are employed to deliver actives transdermally. They have been exploited to a large extent in the field of transdermal and targeted delivery of drugs and therapeutic agents in the field of medicine. Liposomes, in the recent past, have also been used in cosmetic formulations such as skin creams.

WO 95/35095 (Yissum Research Development Company of the Hebrew University of Jerusalem) describes a cosmetic or medical composition for topical application to the skin comprising a phospholipid, a lower aliphatic alcohol of two to four carbon atoms and optionally a glycol and at least 20% water. This publication teaches delivery of active ingredients which may have medicinal properties or cosmetic benefits like anti-aging, tanning among others.

US 2002/0012647 (Cannell et al) describes a composition comprising at least one organic phospholipid capable of forming bilayers in aqueous solution, at least one amphoteric surfactant present in an amount greater than the phospholipid and at least one non-ionic surfactant present in an amount greater than the phospholipid, wherein the combined amounts of the essential ingredients is such as to allow at least one water-insoluble ingredient selected from waxes, unneutralised and partially neutralised water-insoluble polymers, resins and latexes to be incorporated into an aqueous solution.

US 6 497 888 (Morancais et al) describes a process, composition and kit for limiting the penetration into the skin and/or other keratinous fibers of at least one cosmetically and/or pharmaceutically active agent. The composition comprises along with the base composition an effective amount of a disperson of vesicles in a medium, the vesicles comprising at least one ceramide of formula: 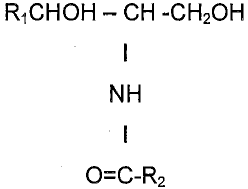 wherein R₁ is chosen from saturated and unsaturated, linear and branched C₁ - C₃₂ alkyl groups and R₂ is chosen from saturated and unsaturated, linear and branched C₁ to C₅₀ alkyl groups.

While many methods and compositions have been described for penetration of skin benefit and skin care actives through liposomes, there exists a need to develop better and more effective methods and compositions to achieve these ends. It is thus an object of the invention to provide for a composition that provides for enhanced delivery of skin benefit agents to the skin. It is another object of the invention to provide for a composition that while providing enhanced delivery of skin benefit agents to the skin, is stable by virtue of being encapsulated in the delivery system of the cosmetic composition.

### Summary of the invention

Thus according to one aspect of the invention there is provided a pro-vesicle for enhanced delivery of skin benefit agents through formation of a vesicular phase in the presence of water in topically applied cosmetic products, according to claim 1.

According to another aspect of the invention there is provided a cosmetic composition for enhanced delivery of skin benefit agents comprising the pro-vesicle of the invention and water such that the weight ratio of water to pro-vesicle is at least 1:1.

According to yet another aspect of the invention there is provided a process for the preparation of the pro-vesicle of the invention comprising the steps of :
(i) preparing a slurry of said benefit agent, said phospholipid, said mono-, di- or tri-ester of glycerol and said straight or branched chain propyl or butyl ester of C₁₄ to C₁₈ fatty acid in a non-aqueous solvent;
(ii) mixing said slurry with the cosmetically acceptable base to form a mixture; and
(iii) separating the non-aqueous solvent from said mixture to form granular pro-vesicle.

### Detailed description of the invention

The first aspect of the invention provides a pro-vesicle for enhanced delivery of skin benefit agents through formation of a vesicular phase in the presence of water in topically applied cosmetic products. By "pro-vesicle" is meant a lipid assembly on a carrier system, which in the presence of water spontaneously provides a vesicular phase. The pro-vesicle comprises the benefit agent to be delivered, a phospholipid, a mono-, di- or tri- ester of glycerol and a straight or branched chain propyl or butyl ester of C₁₄ to C₁₈ fatty acid and a cosmetically acceptable base. The benefit agent to be delivered may be a hydrophobic or hydrophilic benefit agent, preferably a skin lightening agent, a sun screen or a UV blocking agent. A suitable example of a skin lightening agent which is delivered by the pro-vesicle of the invention is niacinamide and a suitable sun screen or UV blocking agent is 2-ethylhexyl-p-methoxycinnamate (Parsol™ MCX) or butylmethoxydibenzoylmethane (Parsol™ 1789). These skin benefit agents are present in an amount in the range of 0.1 to 40% by weight of the pro-vesicle.

### The phospholipid

The phospholipid of the invention is derived from a lecithin, which could be from any source, preferably from soy lecithin. It is preferred that the lecithin comprises at least 32% phospholipid. The phospholipid is preferably present in an amount in the range of 0.5 to 50% by weight of the pro-vesicle.

### Mono-, di- or tri- ester of glycerol

Another essential component of the pro-vesicle of the invention is a mono-, di- or tri- ester of glycerol, preferably a monoester of glycerol. The glycerol is preferably esterified with a fatty acid having 14 to 20 carbon atoms, more preferably 16 to 18 carbon atoms which may be saturated or unsaturated. Thus the preferred glycerol esters are glycerol monostearate, glycerol monooleate or glycerol monopalmitate of which glycerol monostearate is the most preferred. The mono-, di- or tri- ester of glycerol is present in an amount in the range of 2 to 25% by weight of the pro-vesicle.

### Propyl or butyl ester of C₁₄ to C₁₈ fatty acid

The pro-vesicle of the invention comprises a straight or branched chain propyl or butyl ester of C₁₄ to C₁₈ fatty acid, preferably an isopropyl ester of C₁₄ to C₁₈ fatty acid. The compounds that are more preferred include isopropyl myristate, isopropyl ester of hydrogenated 12-hydroxystearic acid or isopropyl palmitate, of which the isopropyl ester of hydrogenated 12-hydroxystearic acid is the most highly preferred compound. The propyl or butyl ester of C₁₄ to C₁₈ fatty acid is preferably present in an amount in the range of 0.5 to 15% by weight of the pro-vesicle.

### Cosmetically acceptable base

The pro-vesicle of the invention comprises a cosmetically acceptable base. A cosmetically acceptable base is a solid material (at ambient temperature) on to which the other components are deposited. Said cosmetically acceptable base is chosen from glucose, sorbitol, talc or stearic acid. The cosmetically acceptable base is present in an amount in the range of 30 to 96% by weight of the pro-vesicle.

### Sterol

The pro-vesicle of the invention optionally comprises a sterol. It has been observed that the inclusion of the sterol in the pro-vesicle of the invention enhances the stability of the pro-vesicle. The sterol may be a phytosterol or a cholesterol, preferably a cholesterol. When present, the sterol is preferably present in an amount in the range of 0.1 to 8% by weight of the pro-vesicle.

### Process for the preparation of the pro-vesicle

The process for the preparation of the pro-vesicle of the invention comprises the steps of:
(i) preparing a slurry of the benefit agent, the phospholipid, the mono-, di- or tri- ester of glycerol and said straight or branched chain propyl or butyl ester of C₁₄ to C₁₈ fatty acid in a non-aqueous solvent;
(ii) mixing said slurry with the cosmetically acceptable base to form a mixture; and
(iii) separating the non-aqueous solvent from said mixture to form granular pro-vesicle.

The slurry is preferably prepared at a temperature greater than 40°C. The non-aqueous solvent is preferably a straight or branched chain alcohol with a carbon chain length of 1 to 4, of which ethanol is the most preferred solvent. The non-aqueous solvent is preferably present in an amount in the range of 5 to 40%, more preferably 10 to 30% by weight of the slurry. The non-aqueous solvent is separated from the mixture by drying preferably under vacuum.

### Cosmetic composition

A cosmetic composition as per the invention comprises the pro-vesicle of the invention and water such that the weight ratio of water to pro-vesicle is at least 1:1, preferably at least 4:1. The cosmetic composition may optionally comprise other cosmetic benefit agents e.g. one or more of emollients, humectants, or thickeners. The cosmetic composition of the invention is prepared by mixing the pro-vesicle of the invention with the water and the other optional ingredients, if present. The pro-vesicle is preferably added to the composition at the end of the mixing preferably at low shear. The temperature at which this process is carried out is preferably in the range of 50 to 70°C.

The cosmetic composition may also comprise other components to act as a diluant, dispersant or carrier for other materials present in the composition, so as to facilitate their distribution when the composition is applied to the skin.

These additional materials, other than water, can include liquid or solid emollients, solvents, humectants, thickeners and powders. Examples of each of these types of additional materials, which can be used singly or as mixtures, are provided hereinbelow.

Emollients are illustrated by but not limited to stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate and myristyl myristate.

Propellants are illustrated by but not limited to propane, butane, isobutane, dimethyl ether, carbon dioxide and nitrous oxide.

Solvents are illustrated by but not limited to ethyl alcohol, isopropanol, acetone, ethylene glycol monoethyl ether, diethylene glycol monobutyl ether and diethylene glycol monoethyl ether.

Powders are illustrated by but not limited to chalk, talc, fullers earth, kaolin, starch, gums, colloidal silica sodium polyacrylate, tetraalkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate.

These additional materials are preferably present at from 10 to 99.9%, preferably from 50 to 99% by weight of the cosmetic composition, and can, in the absence of other cosmetic adjuncts, form the balance of the composition.

### Optional skin benefit agents

Skin lightening ingredients can be advantageously included in the composition to provide skin lightening effects, other than as provided through the pro-vesicle of the invention. These may include vitamin B6, vitamin C, vitamin A or their precursors and mixtures. An especially preferred additional vitamin is vitamin B6. Other skin lightening actives known in the art can also be employed in the invention. Non-limiting examples of skin lightening actives useful herein include aloe extract, ammonium lactate, azelaic acid, kojic acid, lactic acid, linoleic acid, magnesium ascorbyl phosphate, 5-octanoyl salicylic acid, 2,4-resorcinol derivatives, 3,5-resorcinol derivatives, salicylic acid, 3,4,5-trihydroxybenzyl derivatives and mixtures thereof. The composition preferably comprises from about 0.1% to about 10%, more preferably from about 0.1% to about 5% by weight, of a skin lightening ingredient.

The composition of the invention may include an effective amount of a sunscreen or sun-block agent, other than that provided through the pro-vesicle of the invention. Organic and inorganic sunscreens/sun-blocks may be suitably employed in the composition. Suitable organic sunscreen agents include 2-ethylhexyl-p-methoxycinnamate, butylmethoxydibenzoylmethane, 2-hydroxy-4-methoxybenzophenone, octyldimethyl-p-aminobenzoic acid and mixtures thereof. A safe and effective amount of sunscreen may be used in the compositions useful in the subject invention. The composition preferably comprises from about 0.1 % to about 10%, more preferably from about 0.1 % to about 5% by weight, of a sunscreen agent.

Inorganic sun-blocks are also preferably used in the present invention. These include, for example, zinc oxide, iron oxide, silica, such as fumed silica, and titanium dioxide. Ultrafine titanium dioxide in either of its two forms, namely water-dispersible titanium dioxide and oil-dispersible titanium dioxide is especially suitable for the invention. Water-dispersible titanium dioxide is ultra-fine titanium dioxide, the particles of which are non-coated or which are coated with a material to impart a hydrophilic surface property to the particles. Examples of such materials include aluminium oxide and aluminium silicate. Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100 nm, preferably 70 nm or less, more preferably from 10 to 40 nm and most preferably from 15 to 25 nm.

Ultrafine titanium dioxide is the preferred inorganic sun-block agent. The total amount of sun block that is preferably incorporated in the composition according to the invention is from 0.1 to 5% by weight of the composition.

### Optional cosmetic ingredients

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants; binders; biological additives; buffering agents; colorants; thickeners; polymers; astringents; fragrance; humectants; opacifying agents; conditioners; exfoliating agents; pH adjusters; preservatives; natural extracts; essential oils; skin sensates; skin soothing agents and skin healing agents.

The invention will now be illustrated with the following non-limiting examples and figures, wherein:
Figure 1 is a transmission electron microscopy image at 80K magnification of the pro-vesicles present in the cream prepared as example 17; and
Figure 2 is a transmission electron microscopy image at 80K magnification of the vesicular phase obtained by addition of the pro-vesicles of the invention to water.

### Examples

The vesicles as listed in table 1 were prepared in accordance with the procedure detailed below. Comparative examples 1-10 are outside the scope of the invention. Examples 11-15 are according to the invention.

The materials (lecithin, the benefit agent niacinamide (1 gram), the esters and sterol) listed under each example were placed in a beaker and mixed with the ethanol to a homogeneous mixture. The mixture was then sprayed on to stearic acid after which it was vacuum dried for 5 hours to prepare the pro-vesicles. The encapsulation efficiency of the niacinamide was determined as follows.

### Efficiency of encapsulation of niacinamide

Each pro-vesicle sample was dispersed in phosphate buffer saline (PBS of pH 7.4) by stirring for 15 minutes at room temperature. The total niacinamide content of the dispersion (A) was measured by HPLC. Part of the dispersion was centrifuged several times until all the solid material was separated. The supernatant was injected into an HPLC column to measure the unencapsulated niacinamide content (B). The encapsulation efficiency (EE) was determined by EE% = (A - B)/ A *100

**Table 1**

| Example No. | Soy-Lecithin (grams) | Surfactant type | Surfactant (grams) | Thatmat (grams) | Ethanol (grams) | Stearic acid (grams) | Sterol (grams) | EE% |
|---|---|---|---|---|---|---|---|---|
| 1 (Comparative) | 5 | - | - | - | 3 | 10 | 0.5 | 8 |
| 2 (Comparative) | 10 | - | - | - | 5 | 15 | 1.0 | 15 |
| 3 (Comparative) | 5 | Span-60 | 4 | - | 3 | 10 | 0.5 | 10 |
| 4 (Comparative) | 10 | Span-60 | 6 | - | 5 | 15 | 1 | 17 |
| 5 (Comparative) | 5 | BDHA | 0.5 | - | 3 | 10 | 0.5 | 5 |
| 6 (Comparative) | 10 | BDHA | 2 | - | 5 | 15 | 1.0 | 12 |
| 7 (Comparative) | 5 | DHP+ CTAB | 0.5+ 0.2 | - | 3 | 10 | 0.5 | 10 |
| 8 (Comparative) | 10 | DHP+ CTAB | 2+ 1 | - | 5 | 15 | 1.0 | 20 |
| 9 (Comparative) | 5 | GMS | 2 | - | 3 | 10 | 0.5 | 18 |
| 10 (Comparative) | 5 | - | - | 2 | 3 | 10 | 0.5 | 15 |
| 11 | 5 | GMS | 2 | 2 | 3 | 10 | - | 40 |
| 12 | 5 | GMS | 2 | 1 | 3 | 10 | 0.5 | 55 |
| 13 | 10 | GMS | 8 | 3 | 5 | 15 | 1.0 | 60 |
| 14 | 5 | GMO | 2 | 1 | 3 | 10 | 0.5 | 45 |
| 15 | 10 | GMO | 8 | 3 | 5 | 15 | 1 | 50 |

- Thatmat:: Isopropyl ester of 12- hydroxystearic acid
- BDHA:: Benzyl dimethyl hexadecyl ammonium chloride
- DHP:: Di-hexadecyl phosphate
- GMS:: Glycerol monostearate
- GMO: Glycerol monooleate
- Span-60:: Sorbitan monostearate (from SD Fine chemicals)
- CTAB:: Cetyl trimethyl ammonium bromide

The data in table 1 indicates that examples outside the scope of the invention (comparative examples 1 to 10) have poor encapsulation efficiencies, in the range of 5 to 20%. However a pro-vesicle prepared as per the invention (example 11) provides for a very high encapsulation efficiency of 40% which displays synergistic behaviour as compared to the encapsulation efficiencies obtained when pro-vesicles are prepared with each of the ingredients taken individually (comparative examples 9 and 10). The encapsulation efficiency is further improved by the inclusion of the optional ingredient which is sterol (example 12). Examples 13 to 15 are other examples within the scope of the invention which display very high encapsulation efficiencies.

### Cosmetic Compositions

Cosmetic compositions were prepared without (comparative example 16) and with the pro-vesicle (example 17) of the invention and the compositions are summarized in table 2. The composition of the pro-vesicle as used in example 17 is given in table 3.

**Table 2**

| Ingredients | Comparative example 16 (wt%) | Example 17 (wt%) |
|---|---|---|
| Stearic Acid | 10.0 | 2.0 and balance 8% through pro-vesicle |
| Glycerine | 1.0 | 1.0 |
| Potassium hydroxide | 0.6 | 0.6 |
| Preservatives, methyl and propyl paraben | 0.3 | 0.3 |
| Other minors | 1.8 | 1.8 |
| Niacinamide | 1.0 | Through pro-vesicle |
| Parsol™ MCX | 0.75 | Through Pro-vesicle |
| Parsol™ 1789 | 0.4 | Through Pro-vesicle |
| Provesicle | Not included | Included |
| Water | To 100 | To 100 |

**Table 3**

| Pro-vesicle ingredients | % by weight of the cosmetic composition |
|---|---|
| Lecithin | 1.2 |
| Thatmat | 0.2 |
| GMS | 1.0 |
| Cholesterol | 0.2 |
| Stearic acid | 8.0 |
| Niacinamide | 1.0 |
| Parsol™ MCX | 0.75 |
| Parsol™ 1789 | 0.40 |
| CTAB | 0.2 |
| Tocopherol Acetate | 0.001 |
| Total | 12.951 |

A Franz diffusion cell experiment was conducted using a pig's back skin model to compare the amount of niacinamide present in the skin after application of the cosmetic compositions of comparative example 16 and example 17. The Franz diffusion cell experiment is described below.

### Franz Diffusion Cell Experiment:

Pig's back skin was used as the model skin for the studies. Freshly available pig's back skin was taken and the epidermis was separated from the dermis while keeping the stratum corneum intact. The epidermis was thoroughly washed with phosphate buffer saline (PBS of pH 7.4). It was placed between a donor and a receptor compartment. The receptor compartment was filled with PBS and the temperature of the receptor was maintained at about 32°C. The example (about 200 mg) was applied on the donor side of skin. After three hours, the receptor solution was collected (W). The donor side of the skin was washed five times with 5ml of PBS (X). The skin was chopped into small pieces and washed four times with 5ml of PBS (Y) and soaked overnight in PBS and methanol (Z). Samples W-Z were analysed for niacinamide content by HPLC.

The analysis indicated that the permeated niacinamide content for comparative example 16, i.e. the sum of the niacinamide contents of samples W and Z, expressed as a percentage of the total amount of niacinamide (W+X+Y+Z) was 5% while that of example 17 gave a niacinamide content of about 20%. The invention thus provides a composition that provides for enhanced delivery of skin benefit agents to the skin.

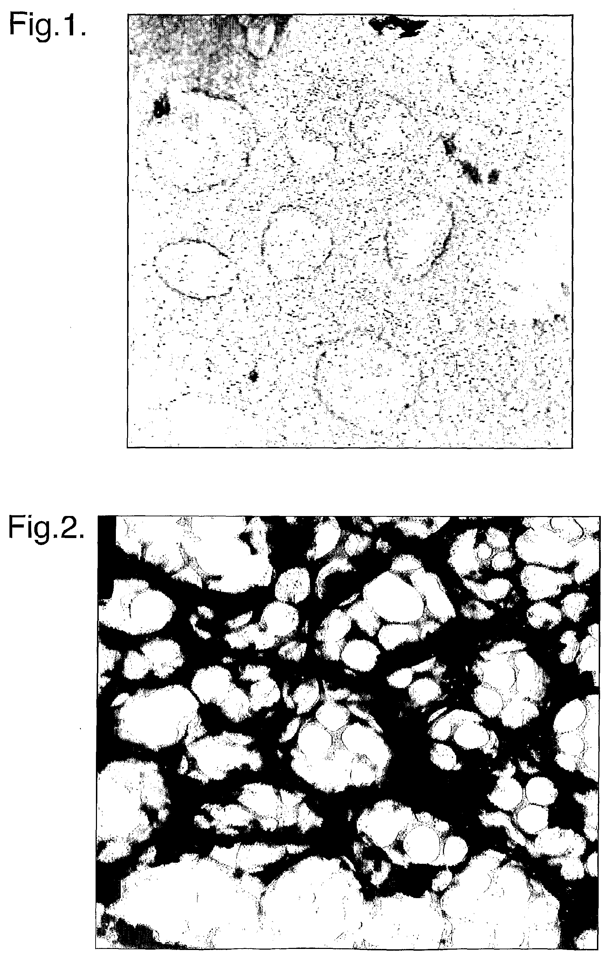

A transmission electron microscopy (TEM) picture at 80K magnification of the provesicles present in example 17 is shown in figure 1 clearly indicating the vesicular phase with an average diameter of about 300 nm. Evidence to show that this vesicular phase can also be prepared by addition of the pro-vesicles to water is provided in figure 2 (also TEM at 80K magnification).

## Claims

1. A pro-vesicle for enhanced delivery of skin benefit agents through formation of a vesicular phase in the presence of water in topically applied cosmetic products, said pro-vesicle comprising:
(i) the benefit agent to be delivered;
(ii) a phospholipid;
(iii) a mono-, di- or tri- ester of glycerol;
(iv) a straight or branched chain propyl or butyl ester of C₁₄ to C₁₈ fatty acid; and
(v) a cosmetically acceptable base,
wherein said cosmetically acceptable base is chosen from glucose, sorbitol, talc or stearic acid and to which components (i) to (iv) are deposited, and
wherein a pro-vesicle is a lipid assembly on a carrier system which in the presence of water spontaneously provides a vesicular phase, the carrier system being in the form of a cosmetically acceptable base, wherein the cosmetically acceptable base is present in an amount in the range of 30 to 96% by weight of the pro-vesicle.

2. A pro-vesicle as claimed in claim 1 wherein the phospholipid is derived from lecithin.

3. A pro-vesicle as claimed in claim 2 wherein the lecithin is soy lecithin.

4. A pro-vesicle as claimed in any one of the preceding claims comprising a saturated or unsaturated C₁₆ to C₁₈ fatty acid ester of glycerol.

5. A pro-vesicle as claimed in claim 4 wherein the fatty acid ester is glycerol monostearate.

6. A pro-vesicle as claimed in any one of the preceding claims comprising isopropyl myristate, isopropyl ester of 12-hydroxystearic acid or isopropyl palmitate.

7. A pro-vesicle as claimed in any one of the preceding claims wherein said skin benefit agent is a skin lightening agent, a sunscreen or a UV blocking agent.

8. A pro-vesicle as claimed in any one of the preceding claims comprising a sterol.

9. A pro-vesicle as claimed in claimed in claim 8 wherein said sterol is cholesterol.

10. A pro-vesicle as claimed in claim 8 or 9 wherein said sterol is present in an amount in the range of 0.1 - 8% by weight of the pro-vesicle.

11. A pro-vesicle as claimed in anyone of the preceding claims wherein said phospholipid is present in an amount in the range of 0.5 - 50% by weight of the pro-vesicle.

12. A pro-vesicle as claimed in anyone of the preceding claims wherein said mono-, di- or triester of glycerol is present in an amount in the range of 2 - 25% by weight of the provesicle.

13. A pro-vesicle as claimed in anyone of the preceding claims wherein said straight or branched chain propyl or butyl ester of C14 to C18 fatty acid is present in an amount in the range of 0.5 -15% by weight of the pro-vesicle.

14. A process for the preparation of a pro-vesicle as claimed in anyone of the claims 1 to 13 comprising the steps of:
(i) preparing a slurry of said benefit agent, said phospholipid, said mono-, di or tri- ester of glycerol and said straight or branched chain propyl or butyl ester of C14 to C18 fatty acid in a non-aqueous solvent;
(ii) mixing said slurry with the cosmetically acceptable base to form a mixture; and
(iii) separating the non-aqueous solvent from said mixture to form granular pro-vesicle.

15. A process as claimed in claim 14 wherein the non-aqueous solvent is ethanol.

16. A process as claimed in claim 14 or claim 15 wherein the non-aqueous solvent in said slurry is present in an amount in the range of 10 - 30% by weight.

## Patentansprüche

1. Ein Pro-Vesikel zur verbesserten Abgabe von Hautvorteilsmitteln durch Bildung einer vesikularen Phase in Gegenwart von Wasser in topisch angewandten kosmetischen Produkten, wobei das Pro-Vesikel umfasst:
(i) das abzugebende Vorteilsmittel,
(ii) ein Phospholipid,
(iii) einen Mono-, Di- oder Tri-Ester von Glycerol,
(iv) einen gerad- oder verzweigtkettigen Propyl- oder Butlyester von C₁₄- bis C₁₈-Fettsäure und
(v) einen kosmetisch annehmbaren Grundbestandteil,
wobei der kosmetisch annehmbare Grundbestandteil unter Glucose, Sorbitol, Talk oder Stearinsäure ausgewählt ist und auf den die Bestandteile (i) bis (iv) abgelagert sind und worin ein Pro-Vesikel eine Lipid-Anordnung auf einem Trägersystem darstellt, das in Gegenwart von Wasser spontan eine vesikulare Phase liefert, wobei das Trägersystem in Form eines kosmetisch annehmbaren Grundbestandteils vorliegt, wobei der kosmetisch annehmbare Grundbestandteil in einer Menge in dem Bereich von 30 bis 96 Gewichts-% des Pro-Vesikels vorliegt.

2. Pro-Vesikel, wie im Anspruch 1 beansprucht, wobei das Phospholipid von Lecithin abgeleitet ist.

3. Pro-Vesikel, wie im Anspruch 2 beansprucht, wobei das Lecithin Soja-Lecithin ist.

4. Pro-Vesikel, wie in irgendeinem der vorhergehenden Ansprüche beansprucht, umfassend einen gesättigten oder ungesättigten C₁₆- bis C₁₈-Fettsäureester von Glycerol.

5. Pro-Vesikel, wie in Anspruch 4 beansprucht, wobei der Fettsäureester Glycerolmonostearat ist.

6. Pro-Vesikel, wie in irgendeinem der vorhergehenden Ansprüche beansprucht, umfassend Isopropylmyristat, Isopropylester von 12-Hydroxystearinsäure oder Isopropylpalmitat.

7. Pro-Vesikel, wie in irgendeinem der vorhergehenden Ansprüche beansprucht, wobei das Hautvorteilsmittel ein Hautaufhellungsmittel, ein Sonnenschutzmittel oder ein UV-Blockierungsmittel darstellt.

8. Pro-Vesikel, wie in irgendeinem der vorhergehenden Ansprüche beansprucht, umfassend ein Sterol.

9. Pro-Vesikel, wie im Anspruch 8 beansprucht, worin das Sterol Cholesterol ist.

10. Pro-Vesikel, wie in Anspruch 8 oder 9 beansprucht, wobei das Sterol in einer Menge in dem Bereich von 0,1 bis 8 Gewichts-% des Pro-Vesikels vorliegt.

11. Pro-Vesikel, wie in irgendeinem der vorhergehenden Ansprüche beansprucht, wobei das Phospholipid in einer Menge in dem Bereich von 0,5-50 Gewichts-% des Pro-Vesikels vorliegt.

12. Pro-Vesikel, wie in irgendeinem der vorhergehenden Ansprüche beansprucht, wobei der Mono-, Di- oder Tri-Ester von Glycerol in einer Menge in dem Bereich von 2-25 Gewichts-% des Pro-Vesikels vorliegt.

13. Pro-Vesikel, wie in irgendeinem der vorhergehenden Ansprüche beansprucht, wobei der gerad- oder verzweigtkettige Propyl- oder Butylester von C₁₄- bis C₁₈-Fettsäure in einer Menge in dem Bereich von 0,5-15 Gewichts-% des Pro-Vesikels vorliegt.

14. Verfahren zur Herstellung eines Pro-Vesikels, wie in irgendeinem der Ansprüche 1 bis 13 beansprucht, umfassend die Schritte von:
(i) Zubereitung einer Aufschlämmung des Vorteilmittels, des Phospholipids, des Mono-, Di- oder Tri-Esters von Glycerol und des gerad- oder verzweigtkettigen Propyl- oder Butylesters von C₁₄- bis C₁₈-Fettsäure in einem nicht-wässrigen Lösungsmittel,
(ii) Mischen der Aufschlämmung mit dem kosmetisch annehmbaren Grundbestandteil, um eine Mischung zu bilden, und
(iii) Abtrennen des nicht-wässrigen Lösungsmittels von der Mischung, um ein körniges Pro-Vesikel zu bilden.

15. Verfahren, wie im Anspruch 14 beansprucht, wobei das nicht-wässrige Lösungsmittel Ethanol ist.

16. Verfahren, wie im Anspruch 14 oder Anspruch 15 beansprucht, wobei das nicht-wässrige Lösungsmittel in der Aufschlämmung in einer Menge in dem Bereich von 10-30 Gewichts-% vorliegt.

## Revendications

1. Pro-vésicule pour la délivrance améliorée d'agents bénéfiques pour la peau par la formation d'une phase vésiculaire en présence d'eau dans des produits cosmétiques appliqués par voie topique, ladite pro-vésicule comprenant :
(i) l'agent bénéfique devant être délivré ;
(ii) un phospholipide ;
(iii) un mono-, di- ou tri-ester de glycérol ;
(iv) un ester de propyle ou butyle à chaîne droite ou ramifiée d'un acide gras en C₁₄ à C₁₈ ; et
(v) une base acceptable sur le plan cosmétique,
où ladite base acceptable sur le plan cosmétique est choisie parmi le glucose, le sorbitol, le talc ou l'acide stérique et sur laquelle les composants (i) à (iv) sont déposés, et
où une pro-vésicule est un assemblage de lipides sur un système de véhicule qui, en présence d'eau, génère spontanément une phase vésiculaire, le système de véhicule étant sous la forme d'une base acceptable sur le plan cosmétique, où la base acceptable sur le plan cosmétique est présente en une quantité dans la plage de 30 à 96 % en poids de la pro-vésicule.

2. Pro-vésicule selon la revendication 1, dans laquelle le phospholipide est dérivé de lécithine.

3. Pro-vésicule selon la revendication 2, dans laquelle la lécithine est la lécithine de soja.

4. Pro-vésicule selon l'une quelconque des revendications précédentes, comprenant un ester d'acide gras en C₁₆ à C₁₈ saturé ou insaturé de glycérol.

5. Pro-vésicule selon la revendication 4, dans laquelle l'ester d'acide gras est le monostéarate de glycérol.

6. Pro-vésicule selon l'une quelconque des revendications précédentes, comprenant le myristate d'isopropyle, un ester isopropylique de l'acide 12-hydroxystéarique ou le palmitate d'isopropyle.

7. Pro-vésicule selon l'une quelconque des revendications précédentes, dans laquelle ledit agent bénéfique pour la peau est un agent éclaircissant la peau, un écran solaire ou un agent bloquant les UV.

8. Pro-vésicule selon l'une quelconque des revendications précédentes, comprenant un stérol.

9. Pro-vésicule selon la revendication 8, dans laquelle ledit stérol est le cholestérol.

10. Pro-vésicule selon la revendication 8 ou 9, dans laquelle ledit stérol est présent en une quantité comprise dans la plage de 0,1-8 % en poids de la pro-vésicule.

11. Pro-vésicule selon l'une quelconque des revendications précédentes, dans laquelle ledit phospholipide est présent en une quantité comprise dans la plage de 0,5-50 % en poids de la pro-vésicule.

12. Pro-vésicule selon l'une quelconque des revendications précédentes, dans laquelle ledit mono-, di- ou tri-ester de glycérol est présent en une quantité comprise dans la plage de 2-25 % en poids de la pro-vésicule.

13. Pro-vésicule selon l'une quelconque des revendications précédentes, dans laquelle ledit ester de propyle ou butyle à chaîne droite ou ramifiée d'un acide gras en C₁₄ à C₁₈ est présent en une quantité comprise dans la plage de 0,5-15 % en poids de la pro-vésicule.

14. Procédé de préparation d'une pro-vésicule selon l'une quelconque des revendications 1 à 13, comprenant les étapes consistant à :
(i) préparer une pâte dudit agent bénéfique, ledit phospholipide, ledit mono-, di- ou tri-ester de glycérol et ledit ester de propyle ou butyle à chaîne droite ou ramifiée d'un acide gras en C₁₄ à C₁₈ dans un solvant non aqueux ;
(ii) mélanger ladite pâte avec la base acceptable sur le plan cosmétique afin de former un mélange ; et
(iii) séparer le solvant non aqueux à partir dudit mélange pour former une pro-vésicule granulaire.

15. Procédé selon la revendication 14, dans lequel le solvant non aqueux est l'éthanol.

16. Procédé selon la revendication 14 ou la revendication 15, dans lequel le solvant non aqueux dans ladite pâte est présent en une quantité comprise dans la plage de 10-30 % en poids.
